# EUROPEAN PATENT SPECIFICATION

(11) **EP 2 089 097 B1**
(45) Date of publication and mention of the grant of the patent: **22.01.2014**
(21) Application number: 06824505.9
(22) Date of filing: 30.11.2006
(51) Int. Cl.: A61N 1/05, A61N 1/36, A61N 1/362, A61N 1/372, A61N 5/06

(54) **DEVICE AND METHOD FOR TREATING CARDIAC TISSUE OF A HEART OF A PATIENT WITH THERAPEUTIC LIGHT USING PHOTOBIOMODULATION**
VORRICHTUNG UND VERFAHREN ZUR BEHANDLUNG VON HERZGEWEBE VOM HERZ EINES PATIENTEN MIT THERAPEUTISCHEM LICHT UNTER VERWENDUNG VON PHOTOBIOMODULATION
DISPOSITIF ET PROCÉDÉ DE TRAITEMENT DU TISSU CARDIAQUE D'UN COEUR D'UN PATIENT AVEC UNE LUMIÈRE THÉRAPEUTIQUE, UTILISANT LA PHOTOBIOMODULATION

(43) Date of publication of application: 19.08.2009
(73) Proprietor: St. Jude Medical AB, 175 84 Järfälla (SE)
(72) Inventor: NORLIN-WEISSENRIEDER, Anna, 181 46 Lidingö (SE); HENRIQUEZ, Leda, 124 32 Bandhagen (SE); STRANDBERG, Hans, 172 65 Sundbyberg (SE); HARSTRÖM, Eva, 165 71 Hässelby (SE); SJÖGREN, Mikael, 740 83 Fjärdhundra (SE); NAESLUND, Annika, 167 64 Bromma (SE); ECKERDAL, Johan, 741 92 Knivsta (SE)
(74) Representative: Sjölander, Henrik
(86) International application number: PCT/SE2006/001375
(87) International publication number: WO 2008/066423

(56) References cited:
- WO-A1-98/43703
- WO-A2-01/03599
- WO-A2-02/100480
- US-A- 6 152 882
- US-A1- 2002 116 029
- US-A1- 2004 260 367
- US-A1- 2005 096 720
- US-A1- 2006 217 793
- US-B1- 6 829 509

## Description

### TECHNICAL FIELD

The present invention generally relates to cardiac pacing systems and, in particular, to methods, implantable medical devices, and a medical lead for treatment of cardiac tissue of a heart of a patient with therapeutic light using an implantable medical device.

### BACKGROUND OF THE INVENTION

In pacemaker applications it is of high importance to design electrode surfaces with good biocompability in order to reduce the inflammatory responses. The amount and character of the inflammation affects the thickness of the scar tissue that encapsulates the electrode as a result of the healing and down regulation of the inflammation responses. Poor healing results in a thick scar tissue and hence the pacemaker is required to supply stimulation pulses of higher energy to be able to stimulate the healthy cardiac cells shielded by the scar tissue. The fact that the electrical field in one location decreases with the square of the distance to the source illustrates the importance to keep the fibrous tissue as thin as possible. Thus, to increase and ensure high efficiency of the pacemaker it is necessary to ensure a thin fibrous capsule formation around the electrode.

US 6,152,882 discloses an apparatus for in vivo chronic measurement of cardiac monophasic action potentials. A sensing electrode is place din contact with cardiac tissue and a referenc electrode is placed in proximity of the sensing electrode. A transient localized depolarization is intermittently induced in cells of the cardiac tissue adjacent to the sensing electrode. The potential difference between the two electrodes is measured.

### BRIEF DESCRIPTION OF THE INVENTION

Thus, an object of the present invention is to provide an implantable medical device for improving the healing of the trauma following the implantation of the cardiac electrode into the cardiac tissue.

Another object of the present invention is to provide an implantable medical device for reducing a stimulation threshold at a contact area of the cardiac electrode.

A further object of the present invention is to provide an implantable medical device for improving regeneration of cardiac cells after an implantation of cardiac electrodes.

These and other objects of the present invention are achieved by means of an implantable medical device having the features defined in the independent claims. Preferable embodiments of the invention are characterized by the dependent claims.

According to an aspect of the present invention there is provided a medical lead that is connectable to an implantable medical device including a pulse generator adapted to produce cardiac stimulating pacing pulses. The medical lead comprises at least one fixation means adapted to fixate the lead at at least one fixation area of cardiac tissue of the patient; at least one electrode for delivering the pulses to cardiac tissue of a heart of a patient when connected to the implantable medical device; and at least one light emitting means adapted to emit therapeutic light, the light emitting means being arranged to emit the therapeutic light towards the at least one fixation area and/or towards at least one contact area of the cardiac tissue where the at least one electrode substantially abuts against the cardiac tissue.

According to a second aspect of the present invention there is provided an implantable medical device including a pulse generator adapted to produce cardiac stimulating pacing pulses. The implantable medical device is connectable to at least one lead comprising electrodes for delivering the pulses to cardiac tissue of a heart of a patient and at least one fixation means adapted to fixate the lead at at least one fixation area of cardiac tissue of the patient. Furthermore, the implantable medical device comprises: at least one light emitting means adapted to emit therapeutic light, the light emitting means being arranged to emit the therapeutic light towards the at least one fixation area and/or towards at least one contact area of the cardiac tissue where the at least one electrode substantially abuts against the cardiac tissue.

There is provided a method for treating cardiac tissue of a heart of a patient with therapeutic light using an implantable medical device including a pulse generator adapted to produce cardiac stimulating pacing pulses. The implantable medical device is connectable to at least one lead comprising electrodes for delivering said pulses to cardiac tissue of a heart of a patient and at least one fixation means adapted to fixate said lead at at least one fixation area of cardiac tissue of said patient. The method comprises the step of emitting therapeutic light using light emitting means towards said at least one fixation area of cardiac tissue and/or towards a contact area of said cardiac tissue where said at least one electrode substantially abut against said cardiac tissue.

There is provided a medical system comprising an implantable medical device according to the second aspect of the present invention and at least one lead according to the first aspect of the present invention.

There is provided a computer program product, directly loadable into an internal memory of an implantable medical device according to the second aspect of the present invention, comprising software code portions for causing the implantable medical device to perform steps in accordance with the method according to above.

The invention utilizes the technique photobiomodulation, also called Low Level Laser Therapy (LLLT), Cold Laser Therapy (CLT), Laser Biomodulation, phototherapy or Laser therapy, wherein certain wavelengths of light at certain intensities are delivered for a certain amount of time. More specifically, the present invention is based on the insight of using such therapeutic light to treat cardiac tissue at an implantation site of a cardiac electrode and/or an electrode contact area of the cardiac tissue. This is founded upon the findings that photobiomodulation has been proven to be a successful therapy in wound healing see, for example, "Effect of NASA light-emitting diode irradiation on wound healing", H. T. Whelan et. al., Journal of Clinical Laser Medicine and Surgery, 19, (2001) p 305. It was also confirmed by Whelan et. al. that the cell growth of various cell types in human and rat could be increased by up to 200 % by irradiation of light of certain wavelengths. Furthermore, it has also been shown, for example, in "Low energy laser irradiation reduces formation of scar tissue after myocardial infarction in rats and dogs", U. Oron, et. al., Circulation, 103, (2001), p296, that light therapy improves the regeneration of the cardiac cells and decreases the scar tissue formation following a myocardial infarction.

Thus, the present invention provides a number of advantages, for example, the healing of the trauma following the implantation of the cardiac electrode into the cardiac tissue can be improved and enhanced. Furthermore, the stimulation threshold at a contact area of the cardiac electrode can be reduced significantly. A further advantage of the present invention is that the regeneration of cardiac cells after an implantation of cardiac electrodes is improved.

According to one embodiment, the light emitting means is at least one light emitting diode. The at least one light emitting diode may arranged at a distal tip portion of the medical lead adjacent to the fixation means or to an electrode. In other embodiments, the light emitting means are arranged at an outer periphery of the medical lead, for example, in proximity of fixation means arranged at the outer periphery, e.g. tines.

In a further embodiment, the medical lead includes at least one optical fibre adapted to conduct light from at least one light source arranged in the implantable medical device such that said conducted therapeutic light emanates from the at least one optical fibre towards at least one fixation area and/or towards at least one contact area between an electrode and cardiac tissue.

According to embodiments of the present invention, measurable indicators of the healing process are monitored and obtained, continuously or regularly, and in one embodiment, a stimulation threshold is measured or calculated. The fact that during the damage and healing time of 1-4 weeks, the threshold value normally first increases significantly from the value at the implantation and then returns to about the initial value. The treatment can be delivered according to predetermined time schedule, utilizing the above mentioned healing process, or based on feedback from the healing process using, for example, the stimulation threshold such that a variation of the measured thresholds indicates a variation of treatment parameters, for example, an intensity of light. The treatment can be completed when the reduction of the threshold values has ceased. For example, average threshold values can be determined and compared periodically with preceding average values (e.g. an average over a 24 hour period) to obtain a trend over the development of the stimulation threshold, i.e. over the healing process. In the beginning, a trend with increasing values will be obtained and, then one or a few peak values will be identified and altered when the average values will start to decrease. When the decline of the trend has come to an end, the treatment will be stopped. The treatment may be more potent during the phase with increasing threshold values and may be maintained or decreased at the identification of the peak value or values. A more potent treatment may be a higher degree of intensity of light or a constant intensity of light but with a changed intermittence, i.e. longer period of light delivery or a more frequent light delivery with a constant period of light delivery.

In one embodiment of the present invention, the light emitting means are activated such that therapeutic light is emitted according to a treatment protocol including treatment parameters comprising one, a number of or all of: emitting intervals of the therapeutic light, intensity of the emitted therapeutic light, wavelength of the emitted light, intermittence of the emitted therapeutic light, or treatment periods. The protocol may thus comprise a predetermined treatment scheme. In an alternative embodiment, the treatment is varied in dependence of one or more treatment response parameters.

According to another embodiment, the at least one treatment response parameter is a stimulation threshold at a contact area between an electrode and the cardiac tissue.

In embodiments of the present invention, the light emitting means are adapted to emit coherent and monochromatic light having a wavelength in the range of 600 nm - 1000 nm. Furthermore, an intensity of 6-50*mW*/*cm²* and a total dosage of about 1-4*J*/*cm*² may be used.

As realized by the person skilled in the art, steps of the methods of the present invention, as well as preferred embodiment thereof, are suitable to realize as a computer program or a computer readable medium.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

### BRIEF DESCRIPTION OF THE DRAWINGS

In the following detailed description, reference will be made to the accompanying drawings, of which:
Fig. 1 schematically shows a pacemaker system including an implantable medical device and medical leads in accordance with the present invention;
Fig. 2a schematically illustrates an embodiment of the implantable medical device according to the present invention;
Fig. 2b schematically illustrates another embodiment of the implantable medical device according to the present invention;
Fig. 3 is a projection view of an embodiment of a medical lead according to the present invention;
Fig. 4 is a cross-sectional view of the medical lead shown in Fig. 3;
Fig. 5 is a circuit diagram illustrating a supply circuit for the diodes of the medical lead shown in Fig. 3 and 4;
Fig. 6 a projection view of another embodiment of a medical lead according to the present invention;
Fig. 7 a projection view of a further embodiment of a medical lead according to the present invention;
Fig. 8 a projection view of yet another embodiment of a medical lead according to the present invention;
Fig. 9 a projection view of still another embodiment of a medical lead according to the present invention;
Fig. 10 a projection view of a further embodiment of a medical lead according to the present invention;
Fig. 11 is high-level flow chart of an embodiment of the method for treating cardiac tissue of a heart of a patient with therapeutic light using an implantable medical device according to the present invention; and
Fig. 12 is a high-level flow chart of another embodiment of the method for treating cardiac tissue of a heart of a patient with therapeutic light using an implantable medical device according to the present invention.

### DETAILED DESCRIPTION OF THE INVENTION

In the following, the present invention will be discussed in the context of medical systems comprising at least an implantable pacemaker such as a bi-ventricular pacemaker, and medical leads such as an atrial lead and a ventricular lead.

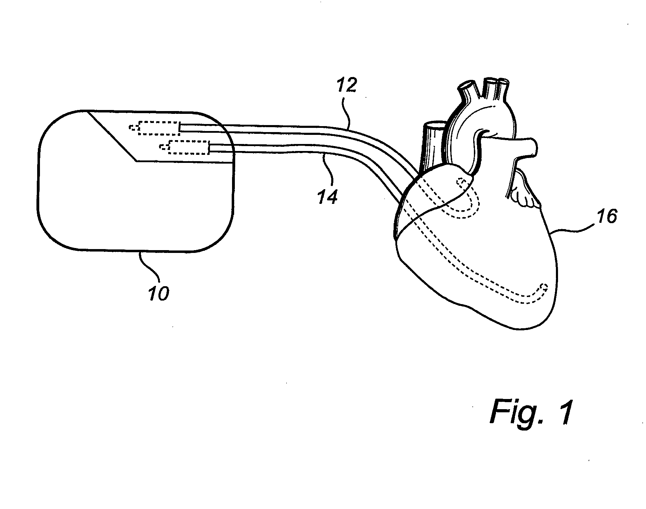

With reference first to Fig. 1, a pacemaker system comprising an implantable bi-ventricular pacemaker 10 connectable to an atrial lead 12 and a ventricular lead 14 including electrodes for providing therapy to a heart 16 of a patient. The leads 12, 14 are implanted into the heart 16 via veins and are fixated at the cardiac tissue by means of, for example, helical screws. As discussed above, the helical screw and/or the electrodes will cause tissue damages, which entail, inter alia, increased stimulation threshold values. This will, in turn, lead to an impaired therapy and an impaired ability to detect heart signals. The healing process of the damaged tissue will eventually improve the therapy, e.g. due to a reduced stimulation threshold.

According to the invention, light emitting means are arranged such that therapeutic light may be emitted at areas of damaged cardiac tissue following an implantation, e.g. at fixation areas of the helical screws and/or at the contact areas of the electrodes, which will be discussed in more detail below.

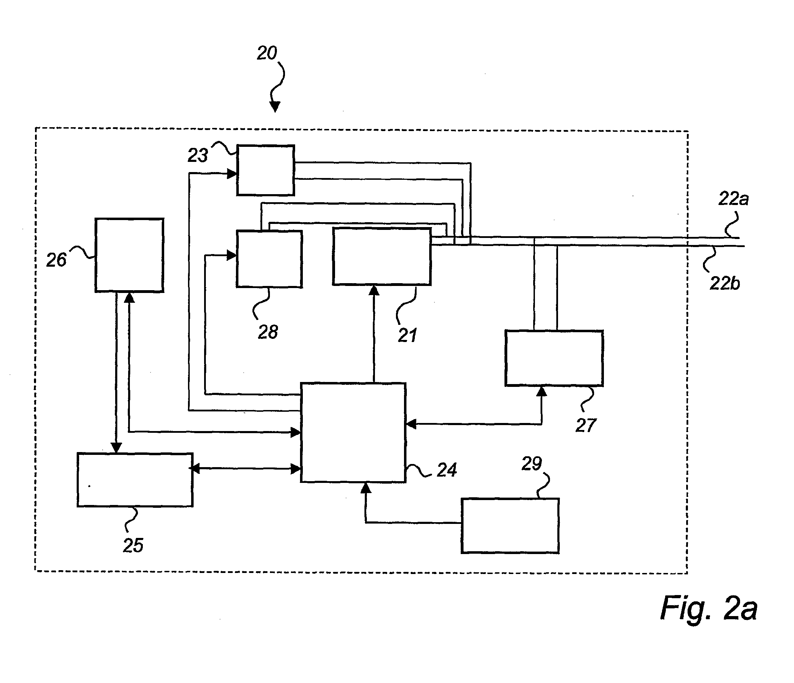

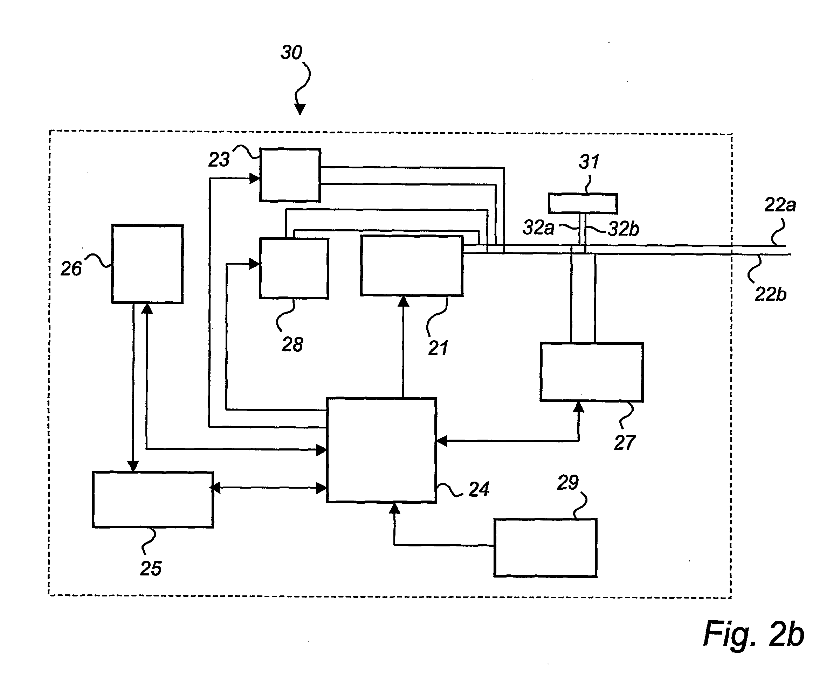

Turning now to Figs. 2a and 2b, an embodiment of an implantable medical device, e.g. a bi-ventricular pacemaker, according to the present invention will be discussed. The implantable medical device 20 comprises a housing (not shown) being hermetically sealed and biologically inert. Normally, the housing is conductive and may, thus, serve as an electrode. The pacemaker 20 is connectable to one or more pacemaker leads, where only two are shown in Figs. 2a and 2b; namely a ventricular lead 22a implanted in the right ventricle of the heart (not shown) and one atrial lead 22b implanted in the right atrium of the heart (not shown).

The leads 22a and 22b can be electrically coupled to the pacemaker 20 in a conventional manner. The leads 22a, 22b comprises one or more electrodes, such as a tip electrode or a ring electrode, arranged to, inter alia, measure the impedance or transmit pacing pulses for causing depolarization of cardiac tissue adjacent to the electrode (-s) generated by a pace pulse generator 21 under influence of a controller or controlling circuit 24 including a microprocessor. The controller 27 controls, inter alia, pace pulse parameters such as output voltage and pulse duration.

Moreover, a storage means 25 is connected to the controller 24, which storage means 25 may include a random access memory (RAM) and/or a non-volatile memory such as a read-only memory (ROM). Storage means 25 is connected to the controller 24 and a signal processing circuit 26. Detected signals from the patient's heart are processed in an input circuit 27 and are forwarded to the controller 24 for use in logic timing determination in known manner.

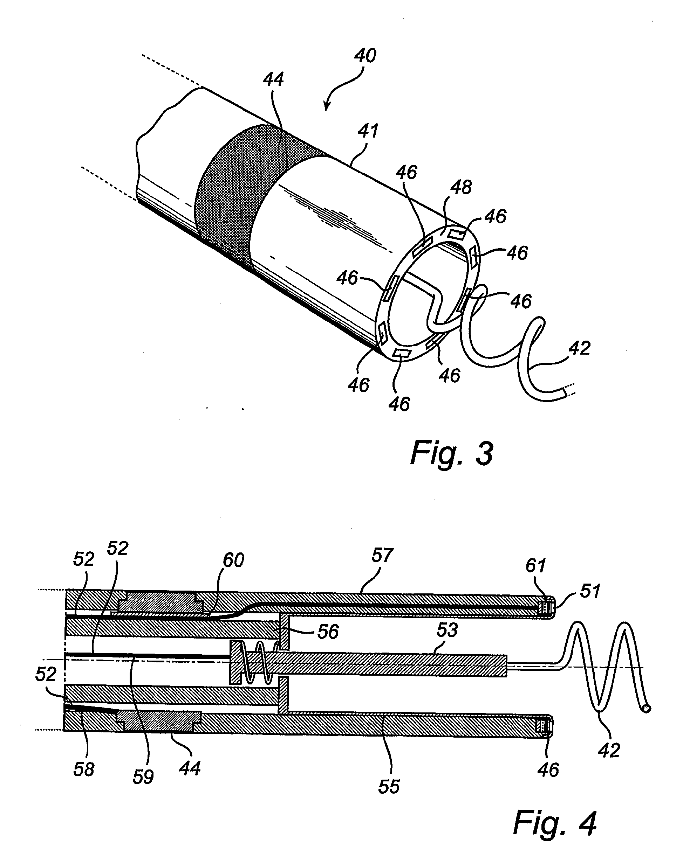

In this embodiment, light emitting means (see, for example, Fig. 3 and 4) is arranged at a distal tip of the leads 22a, 22b and is connected to the controller 24. In one embodiment, the light emitting means in form of light emitting diodes are arranged at peripheries of the ends of tube-shaped leads. The light emitting diodes are adapted to emit monochromatic light having a wavelength of 600 - 1000 nm. The light emitting diodes are connected to a therapy circuit 23 adapted to activate the diodes under control of the controller 24. After implantation, the tube-shaped ends will abut the cardiac tissue and emitted light from the diodes will penetrate into the cardiac tissue at and around the electrode tips. The controller 24 is hence adapted to activate the diodes via the therapy circuit 23 according to a treatment protocol, which may include predetermined or adjustable treatment parameters such as emitting intervals of the therapeutic light, intensity of the emitted therapeutic light, wavelength of the emitted light, and intermittence of the emitted therapeutic light.

Furthermore, a stimulation threshold determining means 28 is arranged in the implantable medical device 20 connected to the electrodes of the leads 22a, 22b. The stimulation threshold determining means 28 is adapted to determine stimulation threshold values of the at least one contact area of the electrodes using pacing responses of at least one applied stimulation pulse. For example, the controller 24 may perform a stimulation threshold test procedure including applying at least one stimulation pulse via at least one the electrodes, for example, a series of stimulation pulses having stepwise increased voltage. The stimulation threshold determining means 28 may measure the pacing responses of the applied stimulation pulses and determine stimulation threshold values of the contact area (-s) of using the pacing responses. Moreover, the stimulation threshold determining means 28 may be adapted to determine an average stimulation threshold value (-s) using pacing responses of series of stimulation pulses applied during a period of time having a predetermined length. This average stimulation threshold value (-s) may be compared with average stimulation threshold values for at least one preceding period of time to determine a trend of the stimulation threshold values, which average stimulation threshold values and/or trend of average stimulation threshold values may be used to adjust treatment parameters of the treatment protocol.

Thus, the stimulation threshold may be used as an indicator of the healing process. During the therapy period, e.g. 1-4 weeks after the implantation, the threshold value will normally increase significantly from the value at the implantation and will eventually decrease to the implantation value. Therefore, a suitable way of identifying when the therapy should be finished is when the reduction of the threshold value has ceased. This is, according to the embodiment of the invention described above, measured by averaging the threshold values over predetermined period of times, for example, over twenty-four hours and is compared with corresponding average values for preceding twenty-four hour periods. In the beginning of the healing process, the values will disclose an increasing trend, i.e. gradually increase over time, and eventually the values will begin to decrease gradually. When the decreasing of the values has ceased, the therapy is determined to be finished.

The therapy parameters can be adjusted during the treatment procedure. For example, a higher light intensity can be used during the increasing trend of the average values and the light intensity can be reduced during the decreasing trend. Alternatively, a constant light intensity but an adjusted intermittence can be utilized, e.g. the periods of light delivery can be adjusted or shorter intervals between the periods of light delivery are used.

The implantable medical device 20 is powered by a battery 29, which supplies electrical power to all electrical active components of the implantable medical device 20 including the light emitting means and stimulation threshold determining means 28. The implantable medical device 20 further comprises a communication unit (not shown), for example, an RF (Radio Frequency) telemetry circuitry for providing RF communications. Thereby, for example, data contained in the storage means 25 can be transferred to an external programmer device (not shown) via the communication unit and a programmer interface (not shown) for use in analyzing system conditions, patient information, etc.

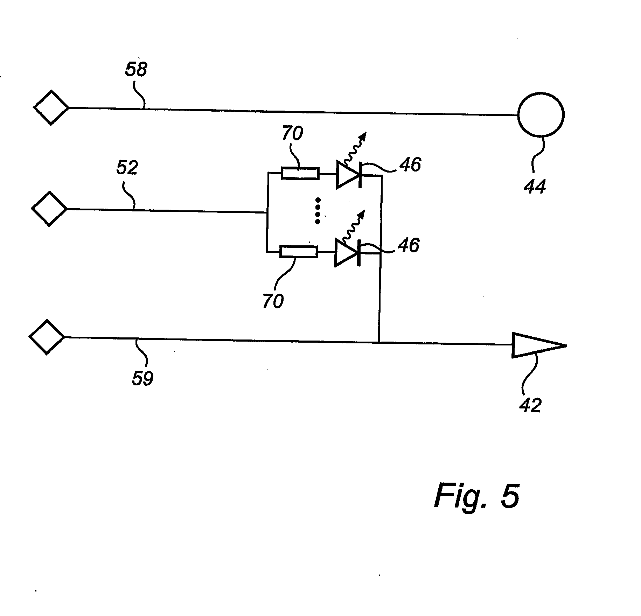

Referring to Fig. 2b, a further embodiment of the implantable medical device according to the present invention will be discussed. Like parts in the implantable medical device shown in Fig. 2a and Fig. 2b will be denoted with the same reference numerals and descriptions thereof will be omitted since they have been described above with reference to Fig. 2a. A light source 31 is arranged in the implantable medical device 30, for example, a laser adapted to emit monochromatic light having a wavelength of 600 - 1000 nm. The light source 31 is connected to a first optical fibre 32a arranged in the ventricular lead 22a and a second optical fibre 32b arranged in the artial lead 22b. The optical fibres 32a, 32b are arranged to conduct light emitted by the light source such that the conducted therapeutic light emanates from the optical fibres 32a, 32b towards at least one fixation area of the fixation means of the cardiac tissue and/or at least one contact area of the cardiac tissue of the at least one electrode. This will be discussed in more detail below with reference to Fig. 5

With reference to Fig. 3, 4, and 5, an embodiment of a medical lead according to the present invention will be discussed. The tube-like medical lead 40, e.g. the ventricular lead 22a or the atrial lead 22b discussed above, comprises a tip screw or helicoidal electrode 42, which may be fixed or extensible, and a ring electrode 44. Furthermore, a number of light emitting diodes 46 are arranged at the periphery 48 of the tube-like end 41 of the lead 40. At an implantation of the lead 40 into the heart of a patient, the helicoidal electrode is screwed into the cardiac tissue and the periphery 48 of the lead will abut against the cardiac tissue. During a treatment procedure when therapeutic light is applied to the tissue, it will penetrate into the tissue substantially at and about the helicoidal electrode 42.

In Fig. 4, a cross-sectional view of the electrode tip of the lead according to the present invention shown in Fig. 3 is illustrated. The tip screw electrode 42 of the lead 40 is, at implantation, screwed in to the cardiac tissue such that the light emitting diodes 46 will abut against the cardiac tissue. In this embodiment, the light emitting diodes 46 are protected from direct contact with body fluids and/or cardiac tissues by means of transparent elements 51, which according to one embodiment may be glass windows. The diodes 46 may be arranged at an angle such that the emitted light is centred towards the helicoidal electrode 42. Alternatively, the transparent elements 51 may be designed as prisms to achieve the centring of the emitted light. Each light emitting diode 46 is supplied with current via an electric conductor 52, which conductors 52, in turn, are connected to the therapy circuit 23.

The helicoidal electrode 42 is attached to a movable shaft 53 and a connector spring 54 is arranged between the shaft 53, a mapping collar 55, and an inner insulating tube 56. An outer insulating tube 57 forms the outer housing of the lead 40. The ring electrode 44 is connected to an electrical conductor 58 and via the conductor 58 to, inter alia, the pace pulse generator 21 and/or the stimulation threshold determining means 28. Furthermore, the helicoidal electrode 42 is connected to an electrical conductor 59 and via the conductor 59 to, inter alia, the pace pulse generator 21 and/or the stimulation threshold determining means 28. The ring electrode 44 is isolated from the electric conductor 52 with an insulation sheet 60.

The light emitting diodes 46 is connected to the mapping collar 55 and to a connection ring 61, which in one embodiment is an electrically conducting rubber flange. The electrically conducting ring 61 has two functions; to mechanically press the diodes 46 such that an adequate electrical connection is achieved and to provide an electrical resistance at the supply to the diodes 46, see Fig. 5, to distribute the current to each of the diodes 46 in an even manner.

The electrical conductors 52, 58, 59 are preferably arranged as helicoidal wires.

With reference to Fig. 5, an embodiment of a connection circuit for the electrodes 42, 44 and light emitting diodes 46. In Fig. 4, one electric conductor 58 to the light emitting diodes was shown. However, each diode 46 could be connected to and supplied via one conductor. In Fig. 5, the diodes 46 are supplied with one conductor 58 via resistance elements 68 for current distribution, for example, 1-100 ohm.

The light emitting diodes are preferably designed to provide a light treatment at an intensity 6-50 *mW*/*cm*² and a total dosage of 1 - 4 *J*/*cm²* per day.

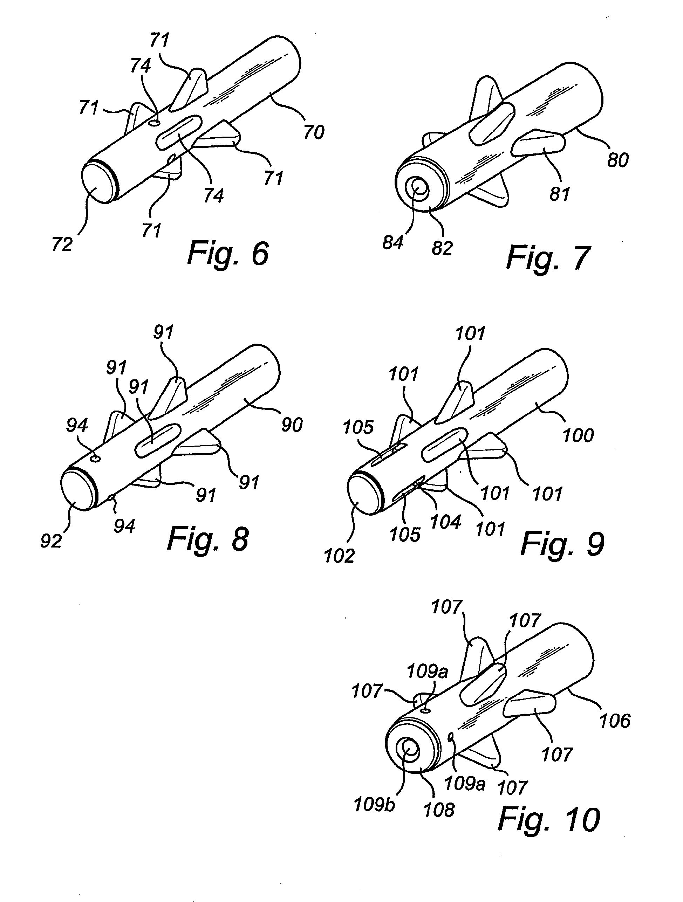

Referring now to Figs. 6-10, further embodiments of the present invention will be discussed. In Fig. 6, a medical lead 70 with a number of tines 71 for fixating the lead 70 at the cardiac tissue is shown. A tip electrode 72 will, after the implantation, abut against the cardiac tissue. Furthermore, a number of light emitting diodes 74 are arranged at the outer surface of the tube-like lead 70 such that emitted therapeutic light can be applied at fixation areas of the tines 71 in the cardiac tissue.

In Fig. 7, a further embodiment of the medical lead according to the present invention is shown. The medical lead 80 is provided with a number of tines 81 for fixating the lead 80 at the cardiac tissue. An annular tip electrode 82 is arranged at the tip of the lead and will, after the implantation, abut against the cardiac tissue. A light emitting diode 84 is arranged at the centre of the tip portion of the lead. When implanted, the light emitting diode can emit therapeutic light at a contact area between the electrode 82 and the cardiac tissue.

In Fig. 8, yet another embodiment of the medical lead according to the present invention is shown. The medical lead 90 is provided with a number of tines 91 for fixating the lead 90 at the cardiac tissue. A tip electrode 92 will, after the implantation, abut against the cardiac tissue. Furthermore, a number of light emitting diodes 94 are arranged at the outer surface or periphery of the tube-like lead 90 such that emitted therapeutic light can be applied or directed at fixation areas of the tines 91 in the cardiac tissue.

According to still another embodiment of the medical lead in accordance with the present invention, see Fig. 9, the medical lead 100 is provided with a number of tines 101 for fixating the lead 100 at the cardiac tissue and a tip electrode 102 that will abut against the cardiac tissue after the implantation. Moreover, a number of light emitting diodes 104 are arranged at the outer surface of the tube-like lead 100. Each diode 104 is arranged in a groove 105 such that emitted therapeutic light can be applied at fixation areas of the tines 101 in the cardiac tissue and at a contact area between the electrode 102 and the cardiac tissue.

Referring now to Fig. 10, yet another embodiment of the present invention will be described. The medical lead 106 is provided with a number of tines 107 for fixating the lead 106 at the cardiac tissue and a tip electrode 108 that will abut against the cardiac tissue after the implantation. Moreover, a number of light emitting diodes 109a are arranged at the outer surface of the tube-like lead 106 and a light emitting diode 109b is arranged at the centre of the tip portion of the lead.

As the person skilled within the art easily realizes, there are a number of conceivable variation to the embodiments described above with reference to Fig. 6-10. For example, the medical lead can be provided with a diode at the centre of the tip portion of the lead and diodes arranged in respective grooves.

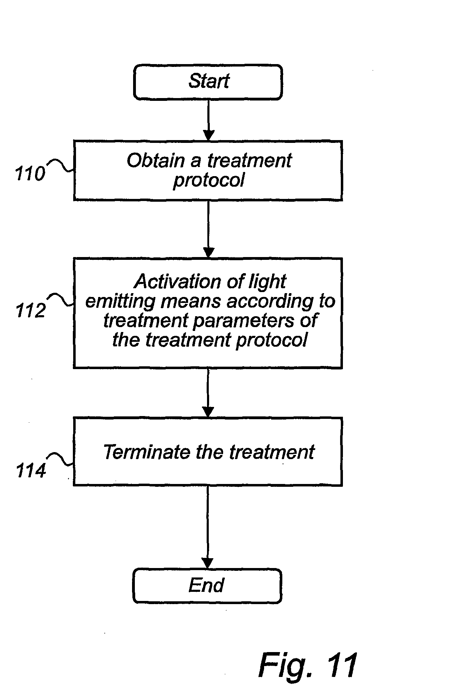

Turning now to Fig. 11, high level flow chart of an embodiment of the method for treating cardiac tissue of a heart of a patient with therapeutic light using an implantable medical device, such as a pacemaker of the type described above, will be discussed.

After a treatment procedure is initiated, the controller 24 obtains, at step 110, a treatment protocol including treatment parameters comprising: emitting intervals of the therapeutic light, intensity of the emitted therapeutic light, wavelength of the emitted light, intermittence of the emitted therapeutic light, and treatment periods. This protocol may be stored in the storage means 25 of the implantable medical device 20, 30. Alternatively, the treatment protocol may be transferred wirelessly to the controller via the communication unit (not shown) from an external programmer workstation. Furthermore, the treatment protocol may be predetermined and/or adapted to the patient. The protocol may also be updated and/or adjusted during a therapy, as will be discussed below, as a response of the applied therapy or upon instructions from, for example, a physician received via the communication unit and the programmer workstation. The therapy may be initiated upon receiving an instruction from the physician received via the communication unit and the programmer workstation.

Thereafter, at step 112, at least one light emitting means, e.g. the light emitting diodes discussed above, is activated in accordance with the treatment protocol. The light therapy is in this embodiment performed in accordance with a predetermined treatment protocol. For example, during a first initial period (e.g. 1-2 weeks), the treatment may be more potent by using a high degree of brightness, or a lower degree of brightness and a changed intermittence, i.e. longer periods of applied light or a more frequent delivery of light with a shorter period of applied light. After this initial period, the treatment can be adjusted during a second period of time (e.g. 1-2 weeks) in that the potency of the therapy is lowered, for example, by using a lower degree of brightness, or a lower degree of brightness and a changed intermittence, i.e. shorter periods of applied light or a more frequent delivery of light with a shorter period of applied light. Subsequently, at step 114, the therapy is terminated.

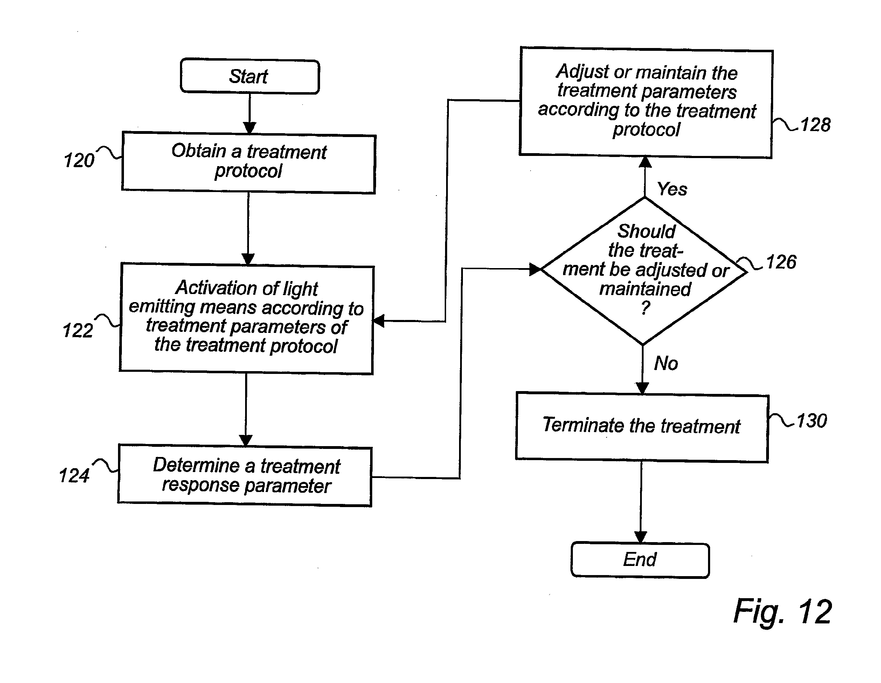

With reference instead to Fig. 12, high level flow chart of another embodiment of the method for treating cardiac tissue of a heart of a patient with therapeutic light using an implantable medical device, such as a pacemaker of the type described above, will be discussed.

After a treatment procedure is initiated, the controller 24 obtains, at step 120, a treatment protocol including treatment parameters comprising: emitting intervals of the therapeutic light, intensity of the emitted therapeutic light, wavelength of the emitted light, intermittence of the emitted therapeutic light, and treatment periods. This protocol may be stored in the storage means 25 of the implantable medical device 20, 30. Alternatively, the treatment protocol may be transferred wirelessly to the controller via the communication unit (not shown) from an external programmer workstation. Furthermore, the treatment protocol may be predetermined and/or adapted to the patient. The protocol may also be updated and/or adjusted during a therapy, as will be discussed below, as a response of the applied therapy or upon instructions from, for example, a physician received via the communication unit and the programmer workstation. The therapy may be initiated upon receiving an instruction from the physician received via the communication unit and the programmer workstation.

Thereafter, at step 122, at least one light emitting means, e.g. the light emitting diodes discussed above, is activated in accordance with the treatment protocol. The light therapy is in this embodiment performed with continuous feedback from healing process. It is continuously checked, at regular intervals, whether a stimulation threshold at a contact area between an electrode and the cardiac tissue satisfies predetermined criteria. Hence, the healing process is continuously monitored in order to adjust the therapy to the healing process such that a variation of measured thresholds provides information whether, for example, the intensity of light or brightness of light should be adjusted or whether the treatment should be terminated.

At step 124, a treatment response parameter is determined or calculated, which in this embodiment is a stimulation threshold. This can be done, for example, by applying at least one stimulation pulse via at least one electrode, measuring pacing responses of the at least one applied stimulation pulse; and determining stimulation threshold values of the at least one contact area of the electrode using the pacing responses. In one embodiment, an average stimulation threshold value is determined using pacing responses of stimulation pulses applied during a period of time having a predetermined length. Furthermore, these average values can be used to determine a trend of the stimulation threshold values over time.

According to one embodiment, initial treatment parameters are used during a period with increasing threshold values, the treatment parameters are adjusted when a decreasing trend has been identified or at the occurrence of a number of peak values, and the treatment is terminated when the decreasing trend has levelled or has come to an end. In a certain embodiment, the parameters of the initial treatment is set such that the treatment is made with a high degree of brightness, or a lower degree of brightness and a changed intermittence, i.e. longer periods of applied light or a more frequent delivery of light with a shorter period of applied light. When a decreasing trend has been identified or at the occurrence of a number of peak values, the treatment is adjusted such that the potency of the therapy is lowered, for example, by using a lower degree of brightness, or a lower degree of brightness and a changed intermittence, i.e. shorter periods of applied light or a more frequent delivery of light with a shorter period of applied light. Finally, the treatment is terminated when the decreasing trend has levelled or has come to an end. Thus, at step 124, an average stimulation threshold value is determined or calculated. Subsequently, at step 126, the present average value is compared with previously calculated threshold to determine the trend. If it is found that the treatment parameters should be adjusted or maintained, for example, in accordance with the description given above, the algorithm proceeds to step 128 where the parameter or parameters is/are adjusted or maintained. Then, the algorithm returns to step 122. On the other hand, if it is found that the treatment should be terminated, the algorithm proceeds to step 130 where the treatment is terminated.

As the person skilled within the art realizes, there are a number of alternative and conceivable variations of the above-described method, for example, in regard to the monitoring of the healing process.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a nonlimiting example thereof and that the scope of protection is defined by the appended patent claims.

## Claims

1. An implantable medical device (10; 20; 30) including:
a pulse generator (21) adapted to produce cardiac stimulating pacing pulses, said device (10; 20; 30) being connectable to at least one lead (12, 14; 22a, 22b; 40; 70; 80; 90; 100; 106) comprising electrodes (42, 44; 72; 82; 92; 102; 108) for delivering said pulses to cardiac tissue of a heart (16) of a patient and at least one fixation means (42; 71; 81; 91; 101; 107) adapted to fixate said lead (12, 14; 22a, 22b; 40; 70; 80; 90; 100; 106) at at least one fixation area of cardiac tissue of said patient,
at least one light emitting means (31, 32; 46; 74; 84; 94; 104; 109a, 109b) adapted to emit therapeutic light, said light emitting means (31, 32; 46; 74; 84; 94; 104; 109a, 109b) being arranged to emit said therapeutic light towards said at least one fixation area and/or towards at least one contact area of said cardiac tissue where said at least one electrode (42, 44; 72; 82; 92; 102; 108) substantially abuts against said cardiac tissue,
a control circuit (23, 24) connected to said light emitting means (31, 32; 46; 74; 84; 94; 104; 109a, 109b), said control circuit (23, 24) being adapted to activate said light emitting means (31, 32; 46; 74; 84; 94; 104; 109a, 109b) to emit said therapeutic light according to a treatment protocol comprises treatment parameters including emitting intervals of said therapeutic light, intensity of said emitted therapeutic light, wavelength of said emitted light, and intermittence of said emitted therapeutic light, **characterized by**:
stimulation threshold determining means (28) connected to at least one electrode (42, 44; 72; 82; 92; 102; 108) and adapted to i) determine an average stimulation threshold value of said at least one contact area of said cardiac tissue using pacing responses of stimulation pulses applied during a period of time having a predetermined length, and ii) compare an average stimulation threshold value for a period of time with average stimulation threshold values for at least one preceding period of time to determine a trend of said average stimulation threshold values, wherein
said control circuit (24) is adapted to obtain said average stimulation threshold values and/or said trend of said average stimulation threshold values from said stimulation threshold determining means (28) and to adjust said treatment parameters based on said obtained average stimulation threshold values and/or said trend of average stimulation threshold values.

2. The implantable medical device according to claim 1, wherein said light emitting means (46; 74; 84; 94; 104; 109a, 109b) is at least one light emitting diode (46; 74; 84; 94; 104; 109a, 109b).

3. The implantable medical device according to claim 2, wherein said at least one light emitting diode (46; 74; 84; 94; 104; 109a, 109b) is arranged at a distal tip portion of said medical lead adjacent to said fixation means (42; 71; 81; 91; 101; 107).

4. The implantable medical device according to claim 2, wherein said at least one light emitting diode (46; 74; 84; 94; 104; 109a, 109b) is arranged at said lead adjacent to said at least one electrode (42, 44; 72; 82; 92; 102; 108).

5. The implantable medical device according to claim 1, wherein said light emitting means (31, 32) comprises at least one light source (31) arranged in said implantable medical device (30) and being adapted to emit said therapeutic light, said light source (31) being connectable to at least one optical fibre (32) arranged in said lead (12, 14; 22a, 22b; 40; 70; 80; 90; 100; 106) such that said light emitted by said at least one light source (31) is conducted and emanated from said at least one optical fibre (31) towards said fixation area and/or towards said at least one contact area.

6. The implantable medical device according to any one of preceding claims, wherein said light emitting means (31, 32; 46; 74; 84; 94; 104; 109a, 109b) is adapted to emit coherent and monochromatic light.

7. The implantable medical device according to any one of preceding claims, wherein said light emitting means (31, 32; 46; 74; 84; 94; 104; 109a, 109b) is adapted to emit light having a wavelength in the range of 600 nm - 1000 nm.

## Patentansprüche

1. Implantierbares Medizinprodukt (10; 20; 30), das Folgendes aufweist:
einen Impulsgenerator (21), der so ausgelegt ist, dass er Herzstimulationserregungsimpulse erzeugt, wobei das Produkt (10; 20; 30) mit mindestens einer Leitung (12, 14; 22a, 22b; 40; 70; 80; 90; 100; 106) verbindbar ist, die Elektroden (42, 44; 72; 82; 92; 102; 108) zum Abgeben der Impulse an Herzgewebe eines Herzens (16) eines Patienten und mindestens ein Befestigungsmittel (42; 71; 81; 91; 101; 107) umfasst, das so ausgelegt ist, dass es die Leitung (12, 14; 22a, 22b; 40; 70; 80; 90; 100; 106) an mindestens einem Befestigungsbereich von Herzgewebe des Patienten befestigt,
mindestens ein lichtaussendendes Mittel (31, 32; 46; 74; 84; 94; 104; 109a, 109b), das so ausgelegt ist, dass es therapeutisches Licht aussendet, wobei das lichtaussendende Mittel (31, 32; 46; 74; 84; 94; 104; 109a, 109b) so angeordnet ist, dass es das therapeutische Licht in Richtung des mindestens einen Befestigungsbereichs und/oder in Richtung von mindestens einem Kontaktbereich des Herzgewebes aussendet, wo die mindestens eine Elektrode (42, 44; 72; 82; 92; 102; 108) im Wesentlichen an das Herzgewebe anstößt,
eine Steuerschaltung (23, 24), die mit dem lichtaussendenden Mittel (31, 32; 46; 74; 84; 94; 104; 109a, 109b) verbunden ist, wobei die Steuerschaltung (23, 24) so ausgelegt ist, dass sie das lichtaussendende Mittel (31, 32; 46; 74; 84; 94; 104; 109a, 109b) ansteuert, damit es das therapeutische Licht gemäß einem Behandlungsprotokoll aussendet, das Behandlungsparameter umfasst, einschließlich Aussendeintervalle des therapeutischen Lichts, Intensität des ausgesendeten therapeutischen Lichts, Wellenlänge des ausgesendeten Lichts und Unterbrechungen des ausgesendeten therapeutischen Lichts, **gekennzeichnet durch**:
Stimulationsschwellenbestimmungsmittel (28), die mit mindestens einer Elektrode (42, 44; 72; 82; 92; 102; 108) verbunden und so ausgelegt sind, dass sie i) einen durchschnittlichen Stimulationsschwellenwert für den mindestens einen Kontaktbereich des Herzgewebes unter Verwendung von Erregungsantworten auf Stimulationsimpulse bestimmen, die während eines Zeitraums mit einer vorher festgelegten Länge angelegt wurden, und ii) einen durchschnittlichen Stimulationsschwellenwert für einen Zeitraum mit durchschnittlichen Stimulationsschwellenwerten für mindestens einen vorangehenden Zeitraum vergleichen, um eine Entwicklung der durchschnittlichen Stimulationsschwellenwerte zu bestimmen, wobei
die Steuerschaltung (24) so ausgelegt ist, dass sie die durchschnittlichen Stimulationsschwellenwerte und/oder die Entwicklung der durchschnittlichen Stimulationsschwellenwerte von den Stimulationsschwellenbestimmungsmitteln (28) erhält, und dass sie die Behandlungsparameter auf der Grundlage der erhaltenen durchschnittlichen Stimulationsschwellenwerte und/oder der Entwicklung durchschnittlicher Stimulationsschwellenwerte anpasst.

2. Implantierbares Medizinprodukt nach Anspruch 1, wobei es sich bei dem lichtaussendenden Mittel (46; 74; 84; 94; 104; 109a, 109b) um mindestens eine Leuchtdiode (46; 74; 84; 94; 104; 109a, 109b) handelt.

3. Implantierbares Medizinprodukt nach Anspruch 2, wobei die mindestens eine Leuchtdiode (46; 74; 84; 94; 104; 109a, 109b) an einem distalen Spitzenabschnitt der medizinischen Leitung angrenzend an dem Befestigungsmittel (42; 71; 81; 91; 101; 107) angeordnet ist.

4. Implantierbares Medizinprodukt nach Anspruch 2, wobei die mindestens eine Leuchtdiode (46; 74; 84; 94; 104; 109a, 109b) an der Leitung angrenzend an der mindestens einen Elektrode (42, 44; 72; 82; 92; 102; 108) angeordnet ist.

5. Implantierbares Medizinprodukt nach Anspruch 1, wobei das lichtaussendende Mittel (31, 32) mindestens eine Lichtquelle (31) umfasst, die in dem implantierbaren Medizinprodukt (30) angeordnet und so ausgelegt ist, dass sie das therapeutische Licht aussendet, wobei die Lichtquelle (31) mit mindestens einem Lichtwellenleiter (32) verbindbar ist, der in der Leitung (12, 14; 22a, 22b; 40; 70; 80; 90; 100; 106) angeordnet ist, sodass das Licht, das von der mindestens einen Lichtquelle (31) ausgesendet wird, von dem mindestens einen Lichtwellenleiter (31) in Richtung des Befestigungsbereichs und/oder in Richtung des mindestens einen Kontaktbereichs geleitet und ausgestrahlt wird.

6. Implantierbares Medizinprodukt nach einem der vorhergehenden Ansprüche, wobei das lichtaussendende Mittel (31, 32; 46; 74; 84; 94; 104; 109a, 109b) so ausgelegt ist, dass es kohärentes und monochromatisches Licht aussendet.

7. Implantierbares Medizinprodukt nach einem der vorhergehenden Ansprüche, wobei das lichtaussendende Mittel (31, 32; 46; 74; 84; 94; 104; 109a, 109b) so ausgelegt ist, dass es Licht mit einer Wellenlänge im Bereich von 600 nm bis 1000 nm aussendet.

## Revendications

1. Dispositif médical implantable (10 ; 20 ; 30) comprenant :
un générateur d'impulsions (21) adapté pour générer des impulsions régulatrices de stimulation cardiaque, ledit dispositif (10 ; 20 ; 30) pouvant être raccordé à au moins une dérivation (12, 14 ; 22a, 22b ; 40 ; 70 ; 80 ; 90 ; 100 ; 106) comprenant des électrodes (42, 44 ; 72 ; 82 ; 92 ; 102 ; 108) pour transmettre lesdites impulsions à du tissu cardiaque d'un coeur (16) d'un patient et au moins un moyen de fixation (42 ; 71 ; 81 ; 91 ; 101 ; 107) adapté pour fixer ladite dérivation (12, 14 ; 22a, 22b ; 40 ; 70 ; 80 ; 90 ; 100 ; 106) à au moins une zone de fixation de tissu cardiaque dudit patient,
au moins un moyen émettant de la lumière (31, 32 ; 46 ; 74 ; 84 ; 94 ; 104 ; 109a, 109b) adapté pour émettre une lumière thérapeutique, ledit moyen émettant de la lumière (31, 32 ; 46 ; 74 ; 84 ; 94 ; 104 ; 109a, 109b) étant agencé pour émettre ladite lumière thérapeutique vers ladite au moins une zone de fixation et/ou vers au moins une zone de contact dudit tissu cardiaque où ladite au moins une électrode (42, 44 ; 72 ; 82 ; 92 ; 102 ; 108) appuie essentiellement contre ledit tissu cardiaque,
un circuit de commande (23, 24) raccordé audit moyen émettant de la lumière (31, 32 ; 46 ; 74 ; 84 ; 94 ; 104 ; 109a, 109b), ledit circuit de commande (23, 24) étant adapté pour activer ledit moyen émettant de la lumière (31, 32 ; 46 ; 74 ; 84 ; 94 ; 104 ; 109a, 109b) pour qu'il émette ladite lumière thérapeutique selon un protocole de traitement comprenant des paramètres de traitement incluant des intervalles d'émission de ladite lumière thérapeutique, l'intensité de ladite lumière thérapeutique émise, la longueur d'onde de ladite lumière émise, et l'intermittence de ladite lumière thérapeutique émise, **caractérisé par** :
des moyens de détermination d'un seuil de stimulation (28) raccordés à au moins une électrode (42, 44 ; 72 ; 82 ; 92 ; 102 ; 108) et adaptés pour i) déterminer une valeur moyenne de seuil de stimulation de ladite au moins une zone de contact dudit tissu cardiaque en utilisant des réponses de régulation à des impulsions de stimulation appliquées pendant une période de temps présentant une longueur prédéterminée, et ii) comparer une valeur moyenne de seuil de stimulation pour une période de temps avec des valeurs moyennes de seuil de stimulation pour au moins une période de temps précédente pour déterminer une tendance desdites valeurs moyennes de seuil de stimulation, dans lequel
ledit circuit de commande (24) est adapté pour obtenir lesdites valeurs moyennes de seuil de stimulation et/ou ladite tendance desdites valeurs moyennes de seuil de stimulation desdits moyens de détermination d'un seuil de stimulation (28) et pour ajuster lesdits paramètres de traitement sur la base desdites valeurs moyennes de seuil de stimulation obtenues et/ou de ladite tendance de valeurs moyennes de seuil de stimulation.

2. Dispositif médical implantable selon la revendication 1, dans lequel ledit moyen émettant de la lumière (46 ; 74 ; 84 ; 94 ; 104 ; 109a, 109b) est au moins une diode électroluminescente (46 ; 74 ; 84 ; 94 ; 104 ; 109a, 109b).

3. Dispositif médical implantable selon la revendication 2, dans lequel ladite au moins une diode électroluminescente (46 ; 74 ; 84 ; 94 ; 104 ; 109a, 109b) est agencée sur une partie de bout distale de ladite dérivation médicale adjacente audit moyen de fixation (42 ; 71 ; 81 ; 91 ; 101 ; 107).

4. Dispositif médical implantable selon la revendication 2, dans lequel ladite au moins une diode électroluminescente (46 ; 74 ; 84 ; 94 ; 104 ; 109a, 109b) est agencée sur ladite dérivation adjacente à ladite au moins une électrode (42, 44 ; 72 ; 82 ; 92 ; 102 ; 108).

5. Dispositif médical implantable selon la revendication 1, dans lequel ledit moyen émettant de la lumière (31, 32) comprend au moins une source lumineuse (31) agencée dans ledit dispositif médical implantable (30) et qui est adaptée pour émettre ladite lumière thérapeutique, ladite source lumineuse (31) pouvant être raccordée à au moins une fibre optique (32) agencée dans ladite dérivation (12, 14 ; 22a, 22b ; 40 ; 70 ; 80 ; 90 ; 100 ; 106) de sorte que ladite lumière émise par ladite au moins une source lumineuse (31) est conduite et émane de ladite au moins une fibre optique (31) vers ladite zone de fixation et/ou vers ladite au moins une zone de contact.

6. Dispositif médical implantable selon l'une quelconque des revendications précédentes, dans lequel ledit moyen émettant de la lumière (31, 32 ; 46 ; 74 ; 84 ; 94 ; 104 ; 109a, 109b) est adapté pour émettre de la lumière cohérente et monochromatique.

7. Dispositif médical implantable selon l'une quelconque des revendications précédentes, dans lequel ledit moyen émettant de la lumière (31, 32 ; 46 ; 74 ; 84 ; 94 ; 104 ; 109a, 109b) est adapté pour émettre de la lumière présentant une longueur d'onde comprise dans la plage de 600 nm à 1000 nm.
